# EUROPEAN PATENT SPECIFICATION

(11) **EP 1 885 817 B1**
(45) Date of publication and mention of the grant of the patent: **11.05.2016**
(21) Application number: 06743889.5
(22) Date of filing: 11.05.2006
(51) Int. Cl.: C09K 11/06, C07D 257/02, C07D 311/86, C07D 335/16, A61K 49/00

(54) **RESPONSIVE LUMINESCENT LANTHANIDE COMPLEXES**
ALS SENSOR GEEIGNETE LUMINISZENTE LANTHANIDKOMPLEXE
COMPLEXES DE LANTHANIDE A REPONSE LUMINESCENTE

(30) Priority: 11.05.2005 GB 0509604; 19.05.2005 GB 0526673; 29.06.2005 GB 0513229
(43) Date of publication of application: 13.02.2008
(73) Proprietor: UNIVERSITY OF DURHAM, Durham, DH1 3LE (GB)
(72) Inventor: YU, Junhua University of Durham, Room GB12, Durham DH1 3UP (GB); PAL, Robert University of Durham, Room GB12, Durham DH1 3UP (GB); PARKER, David Universityof Durham, Room GB12, Durham DH1 3UP (GB)
(74) Representative: McNab, Donald C.
(86) International application number: PCT/GB2006/001718
(87) International publication number: WO 2006/120444

(56) References cited:
- WO-A-00/01663
- WO-A-03/035655
- PARKER D; YU J: "A pH-insensitive, ratiometric chemosensor for citrate using europium luminescence" CHEMICAL COMMUNICATIONS, vol. 2005, 20 May 2005 (2005-05-20), pages 3141-3143, XP002389776
- ATKINSON P; FINDLAY K S; KIELAR F; PAL R; PARKER D; POOLE R A; PUSCHMANN H; RICHARDSON S L; STENSON P A; THOMPSON A L; YU J: "Azaxanthones and azathioxanthones are effective sensitisers for europium and terbium luminescence" ORGANIC & BIOMOLECULAR CHEMISTRY, vol. 4, 23 March 2006 (2006-03-23), pages 1707-1722, XP002389777
- YU J; PARKER D: "Synthesis of a Europium Complex for Anion-Sensing Involving Regioselective Substitution of Cyclen" EUROPEAN JOURNAL OF ORGANIC CHEMISTRY, vol. 2005, 25 August 2005 (2005-08-25), pages 4249-4252, XP002389778
- BRETONNIÈRE Y; CANN M J; PARKER D; SLATER R: "Ratiometric probes for hydrogencarbonate analysis in intracellular or extracellular environments using europium luminescende" CHEMICAL COMMUNICATIONS, vol. 2002, 30 July 2002 (2002-07-30), pages 1930-1931, XP002389779
- AIME S; BATSANOV A S; BOTTA M; HOWARD J A K; LOWE M P; PARKER D: "Structure and relaxivity of macrocyclic gadolinium complexes incorporating pyridyl and 4-morpholinopyridyl substituents" NEW JOURNAL OF CHEMISTRY, vol. 23, 1999, pages 669-670, XP002389780
- BRUCE J I; DICKINS R S; GOVENLOCK L J; GUNNLAUGSSON T; LOPINSKI S; LOWE M P; PARKER D; PEACOCK R D; PERRY J J B; AIME S; BOTTA M: "The Selectivity of Reversible Oxy-Anion Binding in Aqueous Solution at a Chiral Europium and Terbium Center: Signaling of Carbonate Chelation by Changes in the Form and Circular Polarization of Luminescence Emission" JOURNAL OF THE AMERICAN CHEMICAL SOCIETY, vol. 122, 26 September 2000 (2000-09-26), pages 9674-9684, XP002389781

## Description

This invention provides luminescent lanthanide complexes of europium and terbium, methods for their efficient sensitisation and their application in time-resolved assays of bioactive species.

### INTRODUCTION

The unique magnetic and spectroscopic properties of the ground and excited states of the f block ions afford considerable scope for the development of new chemical entities that can be used as imaging probes, as components of optoelectronic devices, or as key sensor materials. Particular advantages off-block ions are their intense, line-like and long-lived luminescence at a range of wavelengths spanning the visible and near infrared (NIR) regions, which permits time-gated rejection of unwanted signals arising from (short-lived) auto-fluorescence from biomolecules. Lanthanide chemistry accordingly plays a key role in such diverse areas as display technology and clinical diagnosis.

There is a need for simple modular synthetic routes that lead to stable emissive systems with tunable photophysical properties and high overall quantum yields (>10% for Eu/Tb in competitive media), that resist photo-fading and bleaching, and can be excited at longer wavelengths to minimise competitive absorption by endogenous molecules or tissue (at the very least, they should obviate the use of quartz optics). Moreover, they should, preferably, allow scope for conjugation to biomolecules, and should, preferably, be compatible with other probes to permit multiplexed imaging. Notwithstanding the burgeoning academic literature (e.g. Verhoeven, Bunzli, Raymond and Sammes (for example BH Bakker et al., Coord. Chem. Rev., 2000, 208, 3; JCG Bunzli & C. Piguet, Chem. Soc. Rev., 2005, 34, 1098; S. Petoud et al., J. Am. Chem. Soc., 2003, 125, 13324; and A. Dadabhoy et al., J. Chem. Soc. Perkin Trans 2, 2000, 2359) reporting the chemistry of new emissive lanthanide(III) complexes or probes, no single molecule meets each of these criteria, and new approaches are required.

Moreover, organic chromophores have been widely used as sensitisers of lanthanide emission. However, very few of those possess a S₁-T₁ energy gap small enough to allow excitation at the longest possible wavelengths without detrimental back energy transfer from the excited state of the metal ion to the sensitiser T₁ state. This is a particularly demanding task for the visibly emitting lanthanides, since their high excited state energies restrict the range of possible sensitisers to those with relatively high triplet state energies. Acridones have been used for this purpose, but in polar media possess an inefficient inter-system crossing step, so that sensitiser fluorescence competes with triplet formation. Y. Bretonniere et al. (Chem. Commun. 2002, 1930-1931) describe examples of acridone-containing macrocyclic europium complexes.

### SUMMARY OF THE INVENTION

We have surprisingly found that the lanthanide complexes described herein (incorporating azaxanthone and azathioxanthone sensititisers) undergo efficient sensitized excitation and can be used in time-resolved assays of bioactive species, especially in signalling the variation in the local concentration of endogenous species such as pH or the citrate anion. In particular, the invention relates to luminescent lanthanide complexes incorporating a xanthone, thiaxanthone sensitising moiety, capable of coordinating to a lanthanide ion by an integral pyridyl group or a related group able to bind to a lanthanide ion. The resulting complex is able to emit light following excitation of the organic sensitising moiety and the emission characteristics are a function of the coordination environment.

Without wishing to be bound by theory, it is believed that whilst azaxanthone and thiaazaxanthone chromaphores have, like the acridones mentioned above, relatively high triplet state energies, they have the benefit of a faster rate of inter-system crossing in polar media, so that ligand fluorescence is much less prevalent. As a result of the small singlet-triplet energy gap, they allow sensitisation of a proximate lanthanide ion in the range 335-420 nm, away from co-absorption by many biomolecules. By engineering these chromophores into well-defined complexes, and using them as efficient sensitisers of lanthanide ions, in particular Eu³⁺ and Tb³⁺ we have developed highly emissive and stable systems.

Viewed from one aspect, therefore the invention provides a luminescent lanthanide complex comprising a lanthanide (III) ion with a xanthone or thiaxanthone sensitising moiety, coordinated to the lanthanide ion by the nitrogen atom of an integral pyridyl group or a related group able to bind the lanthanide ion, the complex being as defined in claim 1. In particular the lanthanide (III) ion is a europium (III) or terbium (III) ion.

Viewed from a further aspect, the invention provides a compound having any one of the formulae 1 and 2. 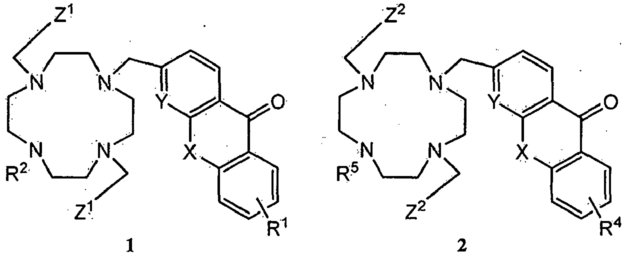 (wherein
R¹ is H, alkyl, aralkyl, CO₂R³, CONHR³;
R² is H, alkyl or aralkyl;
R³ is alkyl, aralkyl or aryl, optionally substituted;
R⁴ is H, alkyl, aralkyl, CO₂R⁶;
R⁵ is CH₂CH₂NHSO₂R⁶;
R⁶ is alkyl, *p*-R⁴-Ph, *p*-OMe-Ph, *p*-CF₃-Ph;
X is O or S;
Y is N, N-oxide or C-OH
Z¹ is CO₂⁻, PR¹O₂⁻, PO₃²⁻, CONHR³; and
Z² is CO₂⁻, PR⁴O₂⁻, PO₃²⁻, CONHR⁴).

In a further aspect of the present invention, the lanthanide complex is able to form a complex with another species, typically an anion. As a result, the emission spectral response of the lanthanide complex is modulated, allowing analysis of the signal by monitoring of two or more emission wavelengths, or by monitoring changes in emission lifetime or circular polarisation.

### BRIEF DESCRIPTION OF THE DRAWINGS

The drawings illustrate selected characterisation data, and exemplify spectral response characteristics under the skilled conditions. In particular:

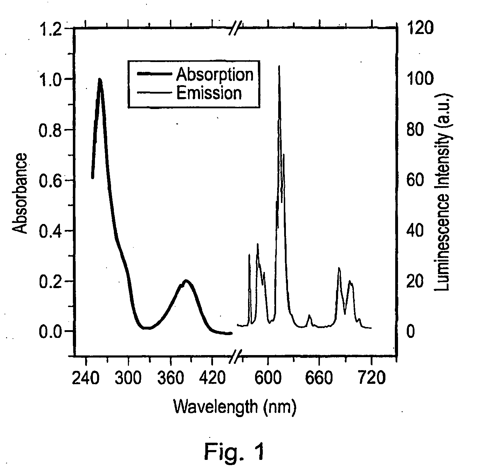

Fig. 1 shows the absorption and luminescence (emission) spectrum of **[EuL¹]³⁺** (see Example 1) in water;

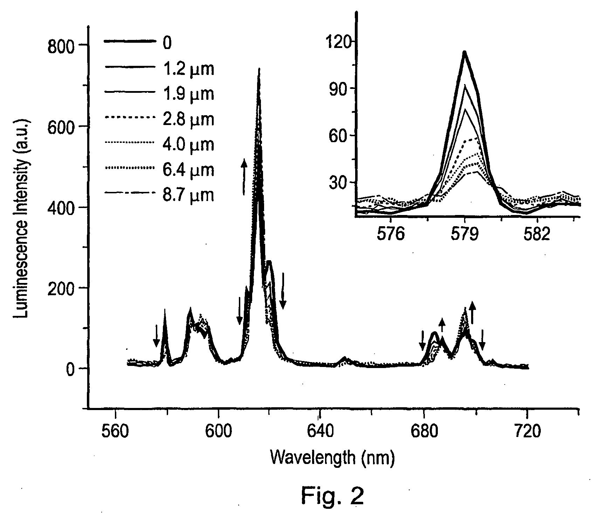

Fig. 2. shows the luminescence spectra and intensity ratio changes of an aqueous solution of **[EuL¹]³⁺** (5 × 10⁻⁶ mol·L⁻¹) upon titration of sodium citrate; λₑₓₑ=384 nm;

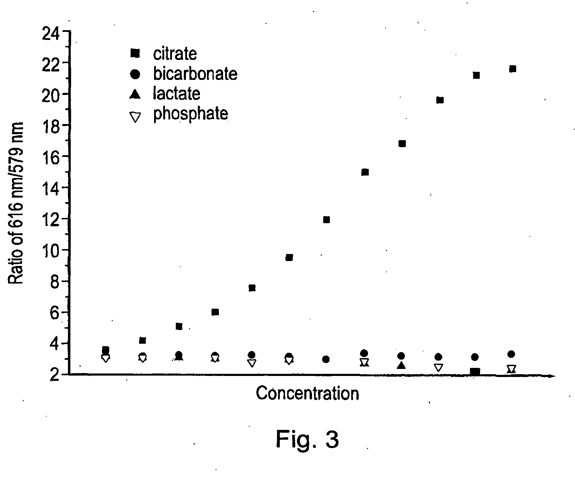

Fig. 3. shows the luminescence intensity ratio of 616 nm to 579 nm of an aqueous solution of **[EuL¹]³⁺** (5 × 10⁻⁶ mol·L⁻¹) upon the titration of citrate, phosphate, bicarbonate, lactate, respectively; excited at 384 nm. Here it shows clearly that, among these anions, only citrate exhibits good affinity to the complex;

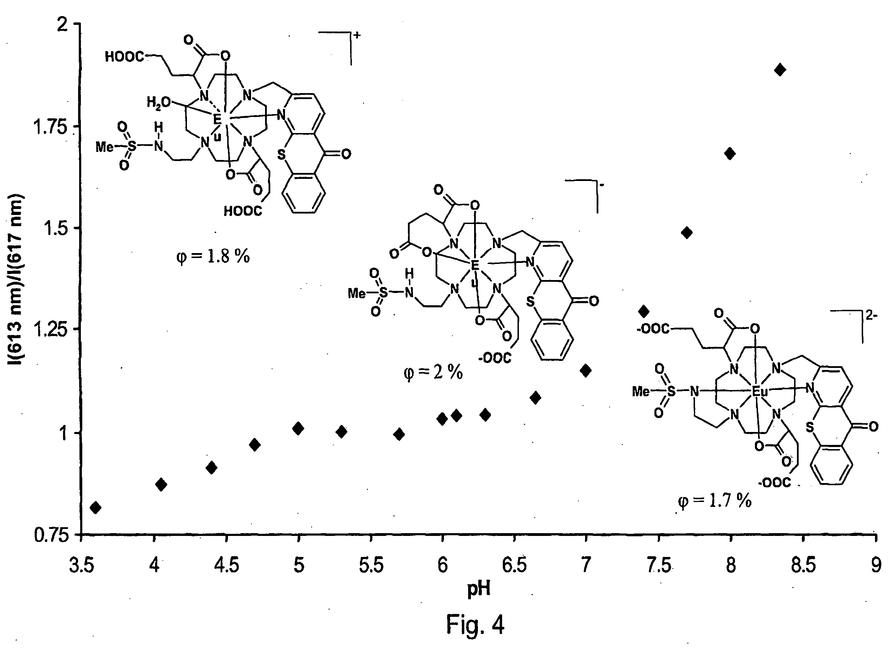

Fig. 4. shows the pH dependence of emission intensity (lumiunescence) ratio of 612 to 618 nm of the Europium complex described in Example 3.

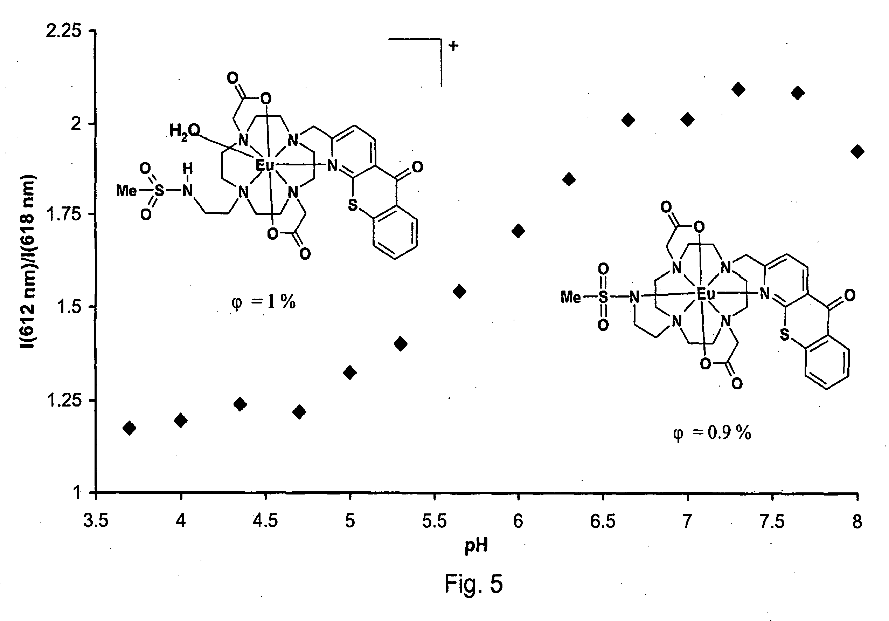

Fig. 5. shows the pH dependence of emission intensity (lumiunescence) ration of 613 to 617 nm of the Europium complex described in Example 4.

### DETAILED DESCRIPTION OF THE INVENTION

The compounds of the invention each comprise xanthone or thiaxanthone sensitising moiety, capable of coordinating to a lanthanide ion by the nitrogen atom of an integral pyridyl group or a related group able to bind a lanthanide ion. The compounds are of the general structures 1 or 2 shown below: 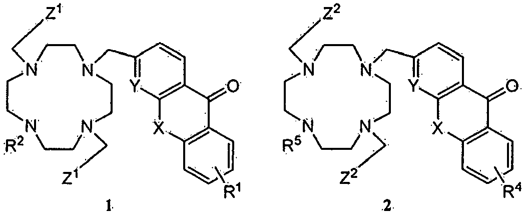 (wherein:
R¹ is H, alkyl, aralkyl, CO₂R³, CONHR³;
R² is H, alkyl or aralkyl;
R³ is alkyl, aralkyl or aryl, optionally substituted;
R⁴ is H, alkyl, aralkyl, CO₂R⁶;
R⁵ is CH₂CH₂NHSO₂R⁶;
R⁶ is alkyl, *p*-R⁴-Ph,*p*-OMe-Ph,*p*-CF₃-Ph;
X is O or S;
Y is N, N-oxide or C-OH
Z¹ is CO₂⁻, PR¹O₂⁻, PO₃²⁻, CONHR³; and
Z² is CO₂⁻, PR⁴O₂⁻, PO₃²⁻, CONHR⁴).

In 1, X may be S or O (preferably S), Y is preferably N, but may also be an N-oxide group or a hydroxy group attached to C, and Z is a carboxylate, phosphinate, phosphonate or carboxamide. The complex is coordinatively unsaturated and is therefore able to bind to an added anion or other electron-rich species, as exemplified in the details given below for [EuL¹] (see Example 1), where Y = N, X, R²= H, Z¹ = (S)-CONHCH(CH₂Ph)CO₂Et and R¹ = H.

In 2, (wherein X and Y are as defined for 1) a substituent on the 12-ring macrocycle, is able to bind reversibly to the lanthanide ion. This coordination is modulated by variations in pH. The alkylsulfonamide substituent R⁶ may be any alkyl or aralkyl group, particularly R = Me, p-tolyl, p-methoxyphenyl or p-trifluoromethylphenyl.

In each of the compounds of the invention, Y is preferably nitrogen and, independently, X is preferably sulfur.

Examples of compounds of the invention, which are described in the Examples which follow below, are 1-(2'-[1-azathioxanthone]methyl)-4,10-*bis*[(S,S)-ethyl-2"-carbamoylmethyl-3-phenylpropanoate]-1,4,7,10-tetra-azacyclododecane, 4-[(1-azathioxanthone)-2-methyl]-1,7-bis(carboxymethyl)-1,4,7,10-tetra-azacyclododecane, 4-[(1-azathioxanthone)-2-methyl]-10-[methylsulfonylamino)ethyl]-1,7-bis (carboxymethyl)-1,4,7,10-tetraazacyclododecane and 4- [(1-azathioxanthone)-2-methyl]-10-[methylsulfonylamino)ethyl]-1,7-bis(α-glutarate)-1,4,7,10-tetraazacyclododecane.

The compounds of the invention may be used to provide luminescent lanthanide complexes by binding to a lanthanide ion. Exemplary of such complexes are those shown below in structures 4 and 5 in which the substituents have the same meanings as set forth in relation to formulae 1 and 2:

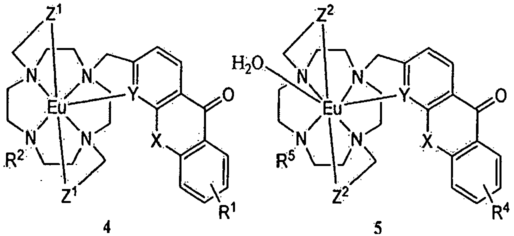

In the structure of the examples shown, the direct coordination of the chromophore to the lanthanide ion minimises the separation between sensitiser and the acceptor lanthanide ion ensuring efficient energy transfer.

The range of anions which may bind includes phosphono-anions, e.g. phosphotyrosine (serine or threonine) sites in peptides or proteins, citrate, lactate, hydrogencarbonate or related species capable of chelating to a lanthanide (III) centre. Selective binding to citrate is preferred. The formulation of further complexes by complexation with other species forms a further aspect of this invention. Viewed from this aspect the invention provides a method of modulating a complex as defined herein comprising reacting the complex with a ligand, typically an anion as defined herein.

In a related aspect, the complex contains a sub-structure that is able to bind reversibly to the lanthanide ion as a function of pH. This intramolecular ligation is signalled by a change in the emission spectral profile of the lanthanide ion, e.g. by changes in two or more emission wavelength intensities or in the lifetime or circular polarisation of emission. In a preferred aspect, an alkylsulfonamide group, linked to the core ligand structure by a carbon-nitrogen bond, is incorporated in the complex structure and reversibly binds to the lanthanide ion over the pH range 3 to 9, especially the range 4.5 to 8. Such behaviour allows the monitoring of local pH using the lanthanide complex as a probe, as required for monitoring local pH changes in real time using ratiometric methods of analysis in analytes ranging from typical in vitro situations to in cellulo or in vivo assays or applications, as required in time-resolved luminescence imaging using microscopy or spectroscopy.

The invention is now illustrated by the following examples, which are not to be considered as limiting of the invention. These describe the synthesis and characterization of representative Eu complexes; the synthetic schemes employed will be evident to those skilled in the art. The figures illustrate the spectral response of certain complexes in the presence of selected anions.

### Example 1: Preparation of 1-(2'-[1-azathioxanthone]methyl)-4,10-bis[(S,S)-ethyl-2"-carbamoylmethyl-3-phenylpropanoate]-1,4,7,10-tetraazacyclododecane (L¹) and europium (III) complex thereof

### (i) Synthesis of 3-Chloromethylpyridothioxanthone:

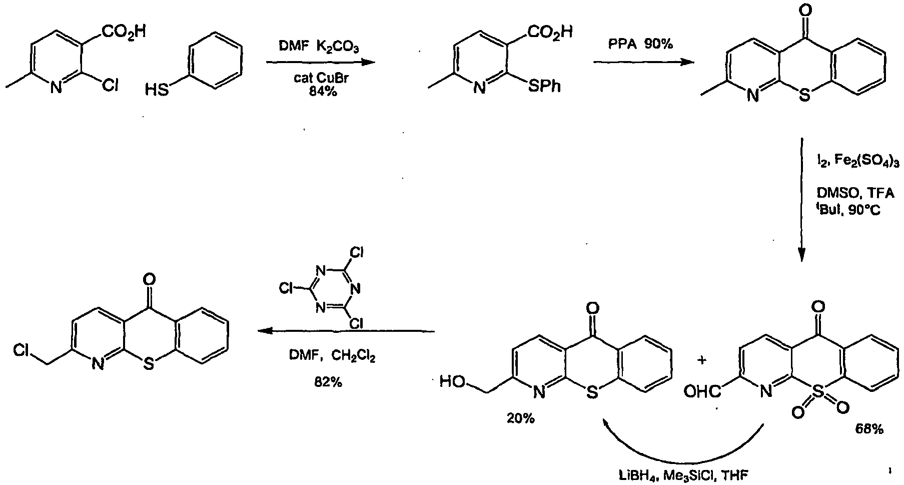

### Synthesis of 6-methyl-2-phenylthionicotinic acid, 1

To 3-chloro-6-methylnicotinic acid (5 g, 29 mmol), thiophenol (3.8 g, 34 mmol, 1.2 eq.) and copper (I) bromide (0.25 g), in DMF (30 mL) was added potassium carbonate (6 g), and the stirred mixture was heated at 130 °C for 15 min. After being heated at 150 °C overnight and cooled down, the mixture (light yellow) was diluted with water (170 mL) and washed with ethyl ether (80 mL x 3). The aqueous solution was acidified with acetic acid to pH 4.5, resulting in a pale yellow precipitate. The solid was filtered, washed with water and dried under reduced pressure to give a yellow solid (5.9 g, 83%). ¹H-NMR (DMSO-d₆): 13.40 (1H, s, -COOH), 8.13 (1H, d, J 7.2, H₁), 7.42-7.52 (5H, m, H_{2'}-H_{6'}), 7.09 (1H, d, J 7.2, H₂), 2.23 (3H, s, CH₃). *m*/*z* (ES⁺): 246 [M + H]⁺, 268 [M + Na]⁺.

### Synthesis of 3-methylpyridothioxanthone, 2

The acid **1** (5.5 g, 22 mmol) and polyphosphoric acid (60 mL) were heated at 120 °C for 4 hrs. The solid dissolved and the solution became brown. The mixture was added to a concentrated aqueous sodium hydroxide solution (300 mL) slowly with vigorous stirring. A precipitate formed. Further sodium hydroxide pellets were added to yield a pH 8 solution. The solution was filtered and the precipitate was washed with water and dried under reduced pressure to give a yellow solid (4.6 g, 90%); m.p. 145-146 °C. ¹H-NMR (DMSO-d₆): 8.73 (1H, d, J 8.2, H₁), 8.60 (1H, d, J 8.4, H₈), 7.65-7.67 (2H, m, H₅+ H₆), 7.48-7.56 (1H, m, H₇),7.31 (1H, d, J 8.2, H₂), 2.70 (3H, s, CH₃). *m*/*z* (ES⁺): 228 [M + H]⁺, 250 [M + Na]⁺, 477 [2M+Na]⁺.

### 3-Hydroxymethyl-4-pyridothioxanthone, 3, and pyridothioxanthen-9-one-10,10-dioxide-3-carboxaldehyde, 4

A solution of 3-methylpyridothioxanthone (3.2 g, 14 mmol), iodine (4.3 g, 17 mmol), iron(III) sulfate pentahydrate (1.2 g, 2.9 mmol), and DMSO (50 mL) was stirred for 5 min, and then tert-butyl iodide (1 mL, 8.7 mmol) and trifluoroacetic acid (3 mL) were added. The mixture was heated at 90 °C for 6 hrs, then cooled to room temperature, following the addition of. aqueous sodium thiosulfate solution (15 mL). The mixture was stirred at r.t. for 1 hr, and then diluted with DCM (400 mL). The solution was washed with water (100 mL × 5), and dried over sodium sulfate anhydrous. The mixture was purified by column chromatography on silica gel, with DCM as the eluant to yield first pyridothioxanthen-9-one-10,10-dioxide-3-carboxaldehyde, then with DCM/methanol (125 : 4) 3-hydroxymethyl-4-pyridothioxanthone, yielding 2.1 g (68%) and 0.7 g (20 %), respectively.

A mixture of lithium borohydride (0.24 g, 12 mmol) and trimethylsilylchloride (1.4 mL, 12 mmol) in 20 mL of THF was added dropwise into a solution of pyridothioxanthen-9-one-10, 10-dioxide-3-carboxaldehyde (1.6 g, 5.9 mmol) in THF in 0 °C. The solution was stirred for a further 2 hrs before adding water (8 mL). The solvents were removed under reduced pressure, giving a yellow solid which was sonicated in a mixture of DCM (300 mL) and 0.5 M hydrochloric acid (100 mL). The organic phase was washed with water, and dried over sodium sulfate. The mixture was purified by column chromatography on silica gel, eluting with DCM/methanol (125 : 4) to yield 3-hydroxymethyl-4-pyridothioxanthone, (0.5 g, 35%).
pyridothioxanthen-9-one-10,10-dioxide-3-carboxaldehyde: ¹H-NMR (CDCl₃): 10.16 (1H, d, *J* 1, CHO), 9.00 (1H, dd, *J* 1.0, 8.0, H₂), 8.62 (1H, d, *J* 8.0, H₈), 8.02 (1H, d, *J* 8.0, H₁), 7.74-7.70 (2H, m, H₅ + H₆), 7.61-7.53 (1H, m, H₇))*. m*/*z* (ES⁺): 296 [M + Na]⁺.
3-hydroxymethyl-4-pyridothioxanthone: ¹H-NMR (CDCl₃): 8.85 (1H, d, *J* 8.2, H₁), 8.62 (1H, d, *J* 10.0, H₈), 7.73-7.66 (2H, m, H₅ + H₆), 7.57-7.53 (1H, m, H₇), 7.41 (1H, d, *J* 10.0, H₂), 4.92 (2H, s). *m*/*z* (ES⁺): 244 [M + H]⁺, 266 [M + Na]⁺, 509 [2M+Na]⁺.

### 3-Chloromethylpyridothioxanthone, 5

A solution of cyanuric chloride (0.29 g, 0.16 mmol) in DMF (1 mL) was stirred for 0.5 hrs, resulting in a white precipitate. Dichloromethane (30 mL) was added, followed by a solution of 3-hydroxymethylpyridothioxanthone (0.25 g, 1.0 mmol) in DCM (20 mL). The solution was stirred at r.t. overnight, then washed with hydrochloric acid (0.5 M, 30 mL) and water, and then dried over sodium sulfate. The mixture was purified by column chromatography on silica gel with DCM/hexane (2:3) as the eluant, yielding 3-chloromethylpyridothioxanthone (0.22 g, 82%). ¹H-NMR (CDCl₃): 8.88 (1H, d, *J* 8.4, H₁), 8.61 (1H, d, *J* 8.1, H₈), 7.73-7.64 (3H, m, H₂ + H₅+ H₆), 7.57-7.52 (1H, m, H₇), 4.76 (2H, s)). *m*/*z* (ES⁺): 262 [M + H]⁺, 284 [M + Na]⁺, 545 [2M+Na]⁺.

### (ii) Synthesis of bromoacetylphenylalanine ethyl ester, 6

Under argon, bromoacetyl bromide (0.89 g, 10.2 mmol) in chloroform (10 mL) was added dropwise to a mixture of chloroform (40 mL), L-phenylalanine ethyl ester (2.3 g, 10 mmol), and triethylamine (3 mL), which was pre-cooled to - 30 °C. The mixture was stirred at r.t. for a further 2 hrs, and then washed with aqueous saturated potassium carbonate solution and water, then dried over sodium sulfate. The solvents were removed under reduced pressure, giving a dark oil, which was dissolved in a mixture of DCM (100 mL) and hexane (300 mL), form which a crystalline solid deposited. The crystals were dried in air, giving colourless needles (2.7 g, 79%). ¹H-NMR (CDCl₃): 7.29 - 7.31 (3H, m, Ar), 7.11 - 7.16 (2H, m, Ar), 4.79 - 4.89 (1H, m, α-H), 4.20 (2H, q, *J* 7.2, **OCH₂**CH₃), 3.86 (2H, s, BrCH₂), 3.16 (2H, d, *J* 5.8, ArCH₂), 1.26 (3H, t, *J* 7.2, OCH₂**CH₃**). *m*/*z* (ES⁺): 336 [M + Na]⁺.

### Synthesis of Cbz-protected cyclen, 7

To a suspension of 1,4,7,10-tetraazacyclododeane (5 g, 29 mmol) and Na₂HPO₄ (14 g) in a mixture of water (50 mL) and dioxane (20 mL), concentrated hydrochloric acid was added to adjust the pH value of the solution to 2.5 - 3.0, and the solid in the solution dissolved. Benzyl chloroformate (12 g, 70 mmol) in dioxane (20 mL) was then added to the above mixture dropwise. The solution was stirred at r.t. for 18 hrs, giving a white precipitate in the mixture. The solvents were removed to yield a white mush which was then washed with 50 mL ethyl ether. Water (100 mL) was added to the mush and the pH was tuned back to neutral with concentrated aqueous sodium hydroxide solution. The above aqueous solution was extracted with ethyl ether (150 mL × 3). The ether was collected, dried over anhydrous sodium sulfate, and evaporated to yield a viscous oil (10 g, 78%). ¹H-NMR (CDCl₃): 7.26 - 7.21 (10H, m, ArH); 5.05 (4H, s, ArCH₂O); 3.33 (8H, br s, cyclen); 2.74 (8H, m, cyclen). *m*/*z* (ES⁺): 441 [M + H]⁺, 463 [M + Na]⁺, 903 [2M+Na]⁺.

### Cbz-protected Phe ester cyclen, 8

Under argon, 7 (2.4 g, 4.5 mmol), N-(2-bromoacetyl) phenylalanine ethyl ester (3.2 g, 10.0 mmol), potassium carbonate (8.5 g), in DMF (50 mL) were stirred at r.t. overnight. Then DCM (200 mL) was added, and the solution was washed with water (100 mL × 10); dried over anhydrous potassium carbonate. The solvents were removed under reduced pressure, resulting in an oily product (4.3 g, 98%). ¹H-NMR (CDCl₃): 7.19 - 7.34 (20H, m, ArH); 5.08 (4H, s, ArCH₂O); 4.81 (2H, m, α-H (Phe)); 4.18 (4H, t, *J* 7.2, O**CH₂**CH₃); 3.32 (8H, br s, cyclen); 3.11 (8H, br s, NCH₂ and ArCH₂C); 2.76 (8H, br s, cycle);1.19 (6H, t, *J* 7.2, OCH2**CH₃**). *m*/*z* (ES⁺): 908 [M+H]⁺.

### Phe ester cyclen, 9

The ester 8 (4.1 g, 4.5 mmol) was dissolved in ethanol (50 mL) and hydrogenated over 0.1 g of Pd/C (20%, w/w) and 40 psi of hydrogen at room temperature for 4 days. The solvents were removed under reduced pressure, giving a colourless oil. The product was re-crystallised from ethyl ether and DCM, yielding 0.7 g light yellow solid product, and 2.0 g of an oily product; the latter slowly solidified, (total 93%). ¹H-NMR (CDCl₃): 7.30 - 7.15 (10H, m, ArH); 4.89 (2H, m, α-H (Phe)); 4.20 (4H, q, *J* 7.2, O**CH₂**CH₃); 3.25 (8H, br s, NCH₂ CO and ArCH₂C); 2.75 (16H, br s, cyclen);1.30 (6H, t, *J* 7.2, OCH2**CH₃**). *m*/*z* (ES⁺): 640 [M + H]⁺.

### Phe ester cyclen pyridothioxanthone: 1-(2'-[1-azathioxanthone]methyl)-4,10-bis[(S,S)-ethyl-2"-carbamoylmethyl-3-phenylpropanoate]-1,4,7,10-tetraazacyclododecane (L¹)

Under argon, **9** (1.5 g, 2.2 mmol), 3-chloromethylpyridoxanthone (0.3 g, 1.1 mmol), and 5 g of potassium carbonate were stirred in acetonitrile (40 mL) at r.t. for 48 hrs. Then DCM (200 mL) was added and the solution was washed with water (80 mL × 3), and dried over sodium sulfate. The product was purified by column chromatography on alumina with DCM/EtOAc/CH3OH (250:15:4) as eluant, yielding a light yellow solid product (0.32 g, 34%). ¹H-NMR (CDCl₃): 8.77 (1H, d, *J* 8.0, H₁), 8.60 (1H, d, *J* 8.0, H₈), 7.71-7.65 (2H, m, H₅ + H₇), 7.59-7.51 (2H, m, H₂ + H₆), 7.31 - 7.12 (10H, m, ArH); 4.81 (2H, m, α-H (Phe)); 4.15 (4H, q, *J* 7.2*,* O**CH₂**CH₃); 3.82 (2H, s, ArCH₂N); 3.26-3.16 (8H, m, cyclen); 3.04 (4H, s, NCH₂CO); 2.82 (4H, br s, cyclen); 2.77 (4H, br s, cyclen); 2.05 (4H, s, ArCH₂C); 1.23 (6H, t, *J* 7.2, OCH2**CH₃**). *m*/*z* (ES⁺): 865 [M + H]⁺;
HRMS (ES⁺), found: 864.4191; C₄₇H₅₈O₇N₇S requires: 864.4197.

### [EuL¹]³⁺

Under argon, ligand 10 (80 mg, 0.09 mmol) and Eu(CF₃SO₃)₃ (134 mg, 0.26 mmol) were boiled under reflux in acetonitrile (3 mL) for 48 hrs. The solution was added slowly to diethyl ether (500mL). The mixture was filtered. The crude product was dissolved in a mixture of DCM (20 mL) and toluene (6 mL). The solution was slowly evaporated at ambient pressure; when most of the DCM had evaporated a precipitate was collected, yielding a grey solid (60 mg, 44%). HRMS (ES⁺), found: 1314.2311; C₄₉H₅₇O₁₃N₇EuF₆S₃ requires: 1314.23

### Example 2: Preparation of 4-[(1-azathioxanthone)-2-methyl]-1,7-bis(carboxymethyl)-1,4,7,10-tetra-azacyclododecane

### (i) Preparation of 4-[(1-Azathioxanthone)-2-methyl]-1,7-bis(tert-butoxycarbonylmethyl)-1,4,7,10-tetraazacyclododecane

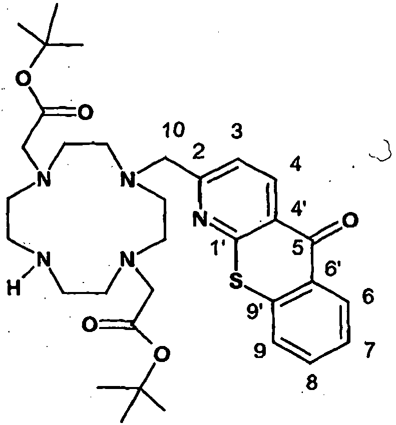

1,7-Bis(tert-butoxycarbonylmethyl)-1,4,7,10-tetraazacyclododecane (250 mg, 0.62 mmol) was combined with 2-bromomethyl-1-azathiaxanthone (1.1 eq., 190 mg) and K₂CO₃ (1 eq., 86 mg) and the mixture stirred in dry MeCN (12 mL) at reflux under argon for 18 h. The reaction was monitored by TLC (DCM : MeOH, 97 : 3) and ESMS⁺ to confirm that the brominated starting material had been consumed. The solvent was removed under reduced pressure. The resulting solid was dissolved in a small volume of DCM (5 mL) and the KBr/K₂CO₃ was filtered out. The crude mixture was purified by column chromatography (DCM→2% MeOH) to yield the title compound as a yellow oil (161 mg, 0.258 mmol, 42%) d_{H} (CDCl₃) 8.71 (1H, H⁴, d, *J* 8.1 Hz), 8.60 (1H, H⁶, d, *J* 8.0 Hz), 7.68 (2H, H^{8,9}, m), 7.49 (1H, H⁷, m), 7.30 (1H, H³, d, J8.1 Hz), 3.87 (2H, H¹⁰, s), 3.13-2.78 (4H, CH₂CO₂ + 16H, NCH₂CH₂N, m), 1.42 (18H, ^{t}Bu, s) d_{c} (CDCl₃) 180.7 (C⁵), 170.2 (CO₂tBu), 161.3 (C²), 158.6 (C^{1'}), 139.2 (C^{4'}), 137.5 (C^{6'}), 133.3 (C⁸), 130.2 (C⁶), 129.3 (C^{9'}), 127.5 (C⁷), 127.1 (C⁹), 124.8 (C⁴), 122.2 (C³), 80.9 (CMe₃), 52.4 (C¹⁰), 57.9 (CH₂CO₂), 50.1, 47.8 (NCH₂ CH₂N), 27.8 (CH₃), m/z (ESMS⁺) 626 (M + 1); R_{f} 0.18 (DCM - 3%MeOH, alumina plate)

### (ii) Preparation of 4-[(1-azathioxanthone)-2-methyl]-1,7-bis(carboxymethyl)-1,4,7,10-tetra-azacyclododecane

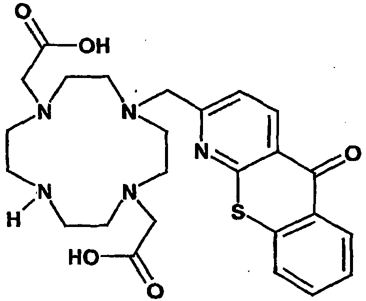

A mixture of trifluoroacetic acid (0.7 mL) and dichloromethane (0.3 mL) was added to 4-[(1-azathioxanthone)-2-methyl]-1,7-bis(tert-butoxycarbonylmethyl)- 1,4,7,10-tetra-azacyclododecane (20 mg, 32 µmol) and the mixture stirred under argon at room temperature for 36 h. The solvents were removed under reduced pressure and a small volume of DCM (3 x 3 mL) was added and removed again under reduced pressure. The crude mixture was dissolved in water (3 mL) and extracted with DCM (3 mL) thrice, and lyophilised to yield the title compound as a pale-orange oil as the trifluoroacetate salt (14 mg, 27 µmol, 86%) which was used in a complexation reaction immediately. *m*/*z* (ESMS⁺) 672 (M+2Na(CF₃COO))

### (iii) Preparation of Eu-Complex

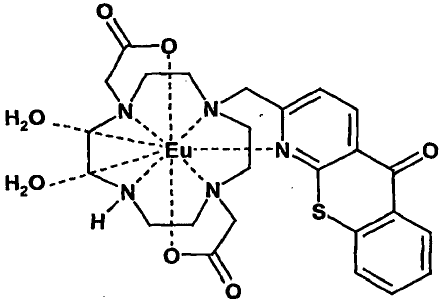

4-[(1-Azathioxanthone)-2-methyl]-1,7-bis(carboxymethyl)-1,4,7,10-tetraazacyclododecane (14mg, 27 µmol) along with Eu(CF₃SO₃)₃ (1.1 eq., 18mg) was dissolved in MeCN (1 mL) and the reaction left stirred at reflux temperature for 18 hrs. After the reaction was cooled to room temperature the solvents were removed under reduced pressure, the remaining residue was dissolved in 5 mL H₂O : MeOH (5 :1). The pH was then adjusted carefully to 10 by addition of conc. NaOH solution (in order to get rid of the Eu-excess as Eu(OH)₃) resulting in a white precipitates removed via a fine syringe filter. The pH was adjusted back to neutral and lyophilised to give the desired complex as a pale yellow solid.

### Example 3: Preparation of of 4-[(1-Azathioxanthone)-2-methyl]-10-[methyl-sulfonylamino)ethyl]-1,7-bis(carboxymethyl)-1,4,7,10-tetraazacyclododecane

### (i) Preparation of 4-[(1-Azathioxanthone)-2-methyl]-10-[methylsulfonylamino)ethyl]-1,7-bis(tert-butoxycarbonylmethyl)-1,4,7,10-tetraazacyclododecane

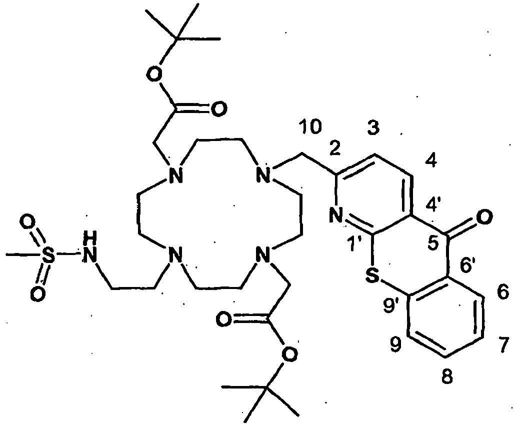

4-[(1-Azathioxanthone)-2-methyl]-1,7-bis(tert-butoxycarbonylmethyl)-1,4,7,10-tetra-azacyclododecane (87 mg, 0.14 mmol) was combined with N-methanesulfonyl-aziridine (1.1 eq., 17.3 mg) and K₂CO₃ (1 eq., 19 mg) stirred in dry MeCN (8 mL) at reflux under argon for 24 h. The reaction was monitored by TLC (DCM : MeOH, 97 : 3) and ESMS⁺ to confirm that the starting secondary amine had been consumed. The solvent was removed under reduced pressure. The resulting solid was dissolved in a small volume of DCM (3 mL) and the K₂CO₃ was filtered out. The crude mixture was purified by column chromatography (DCM→2% MeOH) to yield the title compound as a light brown oil (72 mg, 97 µmol, 69%). d_{H} (CDCl₃) 8.71 (1H, H⁴, d, *J* 8.0 Hz), 8.60 (1H, H⁶, d, *J* 8.0 Hz), 7.68 (2H, H^{8,9}, m), 7.49 (1H, H⁷, m), 7.31 (1H, H³, d, J8.0 Hz), 3.90 (2H, H¹⁰, s), 3.13-2.78 (4H, CH₂CO₂ + 16H, NCH₂CH₂N + 4H, SO₂NHCH₂CH₂N m),), 2.01 (3H, SO₂CH₃), 1.41 (18H, ^{t}Bu, s) d_{c} (CDCl₃) 180.7 (C⁵), 170.2 (CO₂tBu), 161.3 (C²), 158.6 (C^{1'}), 139.2 (C^{4'}), 137.5 (C^{6'}),133.3 (C⁸), 130.2 (C⁶), 129.3 (C^{9'}), 127.5 (C⁷), 127.1 (C⁹), 124.8 (C⁴), 122.2 (C³), 81.1 (CMe₃), 67.0, 67.8 (SO₂NHCH₂CH₂N) 52.4 (C¹⁰), 57.9 (CH₂CO₂), 50.1, 47.8 (NCH₂ CH₂N), 38.5 (SO₂CH₃), 27.8 (C(CH₃)₃), *m*/*z* (ESMS⁺) 746 (M + 1), 747 (M+2), 768 (M+Na); R_{f} 0.44 (DCM : MeOH, 97 : 3, alumina plate)

### (ii) Preparation of 4-[(1-Azathioxanthone)-2-methyl]-10-[methylsulfonylamino)ethyl]-1,7-bis (carboxymethyl)-1,4,7,10-tetraazacyclododecane

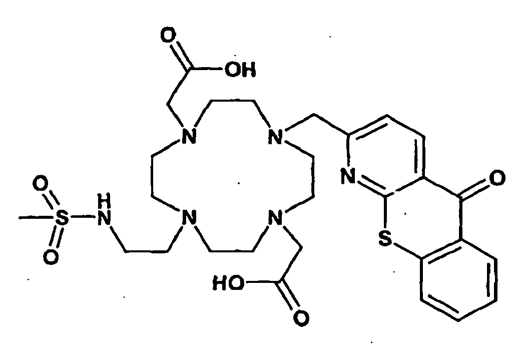

A mixture of trifluoroacetic acid (1.5 mL) and DCM (0.5 mL) was added to 4-[(1-azathioxanthone)-2-methyl]-10-[methylsulfonylamino)ethyl]-1,7-bis(tertbutoxy-carbonylmethyl)-1,4,7,10-tetraazacyclododecane (72 mg (97 µmol) and the reaction stirred under argon at room temperature for 28 h. The solvents were removed under reduced pressure and a small volume of DCM (3 x 3 mL) was added and removed again under reduced pressure. The crude mixture then had been dissolved in water (5 mL) and extracted with DCM (5 mL) thrice, and lyophilised to yield the *title compound* as a dark orange oil which slowly crystallised (55 mg, 87 µmol, 90%). This material was used in a complexation reaction immediately, *m*/*z* (ESMS⁺)658 (M+2Na), m.p. 120-1 °C

### (iii) Preparation of Eu-Complex

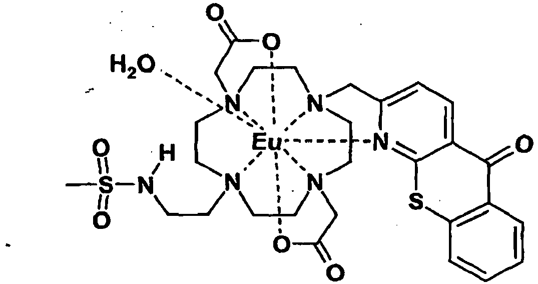

4-[(1-Azathioxanthone)-2-methyl]-10-[methylsulfonylamino)ethyl]-1,7-bis (carboxymethyl)-1,4,7,10-tetraazacyclododecane (28 mg, 44 µmol) was added to Eu(CF₃SO₃)₃ (1.1 eq., 26 mg) and the solids dissolved in a MeCN (2 mL) and the reaction left stirred at reflux temperature for 30 hrs. After the reaction was cooled to room temperature the solvents were removed under reduced pressure, the remaining residue was dissolved in 5 mL water : MeOH (5 :1). The pH was then adjusted carefully to 10 by addition of conc. NaOH solution (in order to get rid of the Eu-excess as Eu(OH)₃) resulting in a white precipitates removed via a fine syringe filter. The pH was adjusted back to neutral and lyophilised to give a light brown solid, which has been loaded onto a DOWEX 1-X8(Cl) anionexchange resin. The column was eluted with water → 10% NH₄OH and the fractions were analysed by ESMS⁺. The fractions were combined and lyophilised to yield the Eu-complex as a light brown powder. *m*/*z* (HRMS⁺) 819,09138 (C₂₈H₃₅O₇N₆S₂EuCl requires 819.09151)

### Example 4: Preparation of 4-1(1-Azathioxanthone)-2-methyl]-10-[methylsulfonylamino)ethyl]-1,7-bis(α-glutarate)-1,4,7,10-tetraazacyclododecane

### (i) Preparation of 1,7-Bis(α-dimethylglutarate)-1,4,7,10-tetraazacyclododecane

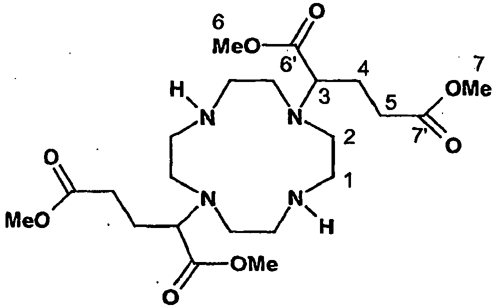

Tetraazacyclododecane (2.00 g, 11.61 mmol), dimethyl-2-bromoglutarate (6.10 g ,25.54 mmol) was dissolved in dry MeCN (20 mL) followed by addition of NaHCO₃ (2.14 g, 2.2 eq.). The mixture was stirred at 55 °C under argon. The reaction was monitored by TLC (DCM : MeOH : NH₄OH 89 : 10 : 1) and ESMS⁺. After 7 days all dimethyl-2-bromoglutarate had been consumed, and the solvent was removed under reduced pressure. The remaining residue was dissolved in DCM (20 mL). The organic layer was washed with HCl (pH 3), dried over K₂CO₃ and the solvents removed under reduced pressure. The residue was purified by column chromatography over silica (DCM : THF : MeOH : NH₄OH, 25 : 65 : 5 : 5). The fractions containing the *title product* were combined and the solvents were removed under reduced pressure to yield a pale brown oil (1.23 g, 2.52 mmol, 21%) d_{H} (CDCl₃) 7.68 (2H, br.s, NH), 3.63 (6H, s, H⁷), 3.57 (6H, s, H⁶), 3.26 (2H, m, H³), 2.78 (16H, m, H^{1,2}), 2.36 (4H, m, H⁵), 1.92 (4H, m, H⁴) d_{c}(CDCl₃) 173.4 (C^{7'}), 172.9 (C^{6'}), 64.1 (C³), 51.9 (CH₃⁶), 51.8 (CH₃⁷), 48.7, 46.5 (CH^{1,2}), 30.0 (C⁵), 22.6 (C⁴), *m*/*z* (ESMS⁺) 489 (M + 1), 490 (M+2), R_{f} 0.32 (DCM: MeOH : NH₄OH, 89 : 10 : 1, silica plate)

### (ii) Preparation of 4-[(1-Azathioxanthone)-2-methyl]-1,7-bis(α-dimethylglutarate)-1,4,7,10-tetraazacyclododecane

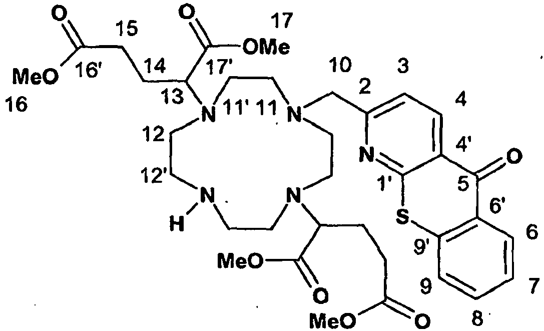

1,7-Bis(α-dimethylglutarate)-1,4,7,10-tetraazacyclododecane (320 mg, 0.66 mmol) was combined with 2-bromomethyl-1-azathiaxanthone (1 eq., 200 mg) and K₂CO₃ (1 eq., 91 mg) and the mixture stirred in dry MeCN (10 mL) at reflux under argon for 30 h. The reaction was monitored by TLC (DCM : MeOH, 97 : 3) and ESMS⁺ to confirm that the brominated starting material had been consumed. The solvent was removed under reduced pressure. The resulting solid was dissolved in a small volume of DCM (5 mL) and the KBr/K₂CO₃ was filtered out. The crude mixture was purified by column chromatography (DCM→2% MeOH) to yield the *title compound* as a pale brown oil (120 mg, 0.168 mmol, 26%) d_{H} (CDCl₃) 8.68 (1H, d, *J* 8.0 Hz, H⁴), 8.43 (1H, m, H⁶), 7.59 (2H, m , H^{8,9}), 7.42 (1H, m, H⁷), 7,24 (1H, d, *J* 8.0 Hz, H³), 3.83 (2H, s, H¹⁰), 3.63 (6H, s, H¹⁶), 3.57 (6H, s, H¹⁷), 3.26 (2H, m, H¹³), 2.97 (16H, m, H^{11,11,12,12'}), 2.36 (4H, m, H¹⁵), 1.92 (4H, m, H¹⁴) d_{c} (CDCl₃) 180.5 (C⁵)173.4 (C^{16'}), 172.9 (C^{17'}),161.4 (C²), 158.6 (C^{1'}), 138.4 (C^{4'}), 137.5 (C^{6'}), 133.3 (C⁸), 130.0 (C⁶), 129.0 (C^{9'}), 127.1 (C⁷), 126.6 (C⁹), 125.3 (C⁴), 122.2 (C³), 65.1 (C¹³), 51.9 (C¹⁷), 51.8 (C¹⁶), 51.3, 50.4, 49.2, 46.5 (C^{11,11,12,12'}), 46.1 (C¹⁰), 30.9 (C¹⁵), 25.7 (C¹⁴), *m*/*z* (ESMS⁺) 714 (M + 1); R_{f} 0.16 (DCM: MeOH, 97 : 3, alumina plate)

### (iii) Preparation of 4-[(1-Azathioxanthone)-2-methyl]-10-[methylsulfonylamino)ethyl]-1,7-bis(α-dimethylglutarate)-1,4,7,10-tetraazacyclododecane

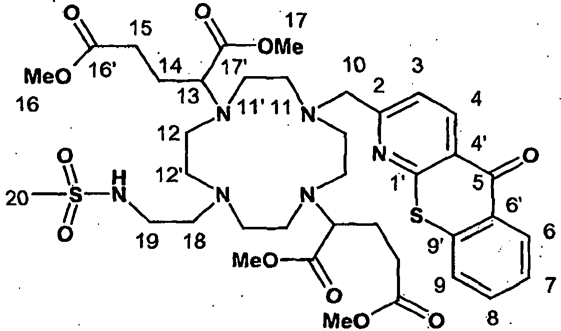

4-[(1-Azathioxanthone)-2-methyl]-1,7-bis(α-dimethylglutarate)-1,4,7,10-tetraazacyclododecane (110 mg, 0.15 mmol) was combined with N-methanesulfonyl-aziridine (1.1 eq., 19.4 mg) and K₂CO₃ (1 eq., 22 mg) stirred in dry MeCN (5 mL) at reflux under argon for 46 h. The reaction was monitored by TLC (DCM : MeOH, 97 : 3) and ESMS⁺ to confirm that the starting material had been consumed. The solvent was removed under reduced pressure. The resulting solid was dissolved in a small volume of DCM (3 mL) and the K₂CO₃ was filtered out. The crude mixture was purified by column chromatography (DCM→2% MeOH) to yield the title compound as a light brown oil which slowly crystallised (50 mg, 60 µmol, 41%). d_{H} (CDCl₃) 8.70 (1H, d, *J* 8.0 Hz, H⁴), 8.48 (1H, m, H⁶), 7.63 (2H, m , H^{8,9}), 7.44 (2H, m, H^{3,7}), 3.83 (2H, s, H¹⁰), 3.63 (6H, s, H¹⁶), 3.60 (6H, s, H¹⁷), 3.26 (2H, m, H¹³), 3.02 (3H, s, H²⁰), 2.93 (16H, m, H^{11,11',12,12'}), 2.50 (6H, m, H^{15,18}), 1.90 (4H, m, H¹⁴), 1.56 (2H, t, *J* 7.8 Hz, H¹⁹) d_{c} (CDCl₃) 180.5 (C⁵) 173.3 (C^{16'}), 173.0 (CH^{17'}), 162.1 (C²), 158.8 (C^{1'}), 138.5 (C^{4'}), 137.5 (C^{6'}), 133.7 (C⁸), 130.0 (C⁶), 128.9 (C^{9'}), 127.2 (C⁷), 126.6 (C⁹), 125.5 (C⁴), 122.7 (C³), 65.9 (C¹³), 51.7 (C¹⁷) 51.4 (C¹⁶), 54.2, 51.3, 49.2, 46.6 (C^{11,11,12,12'}) 46.1 (_{C}¹⁰), 38.1 (C²⁰) 33.4 (C¹⁸), 30.9 (C¹⁵), 25.7 (C¹⁴), 22.9 (C¹⁹), *m*/*z* (ESMS⁺) 835 (M + 1); R_{f} 0.39 (DCM : MeOH, 97 : 3, alumina), m.p. 137-9 °C

### (iv) Preparation of 4-[(1-Azathioxanthone)-2-methyl]-10-[methylsulfonylamino)ethyl]-1,7-bis(α-glutarate)-1,4,7,10-tetraazacyclododecane

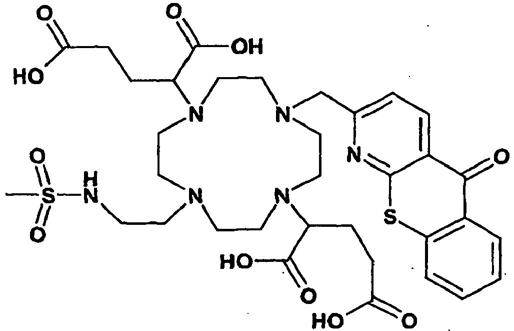

Freshly made 0.1 M KOD (2.5 mL) was added to 4-[(1-Azathioxanthone)-2-methyl]-10-[methylsulfonylamino)ethyl]-1,7-bis(α-dimethylglutarate)-1,4,7,10-tetraazacyclododecane (50 mg (60 µmol) the reaction had left under argon at room temperature and been monitored by NMR. After 3 h. no protecting methyl group signals were observed in the ¹H-NMR spectrum The pH of the mixture was neutralised (pH ≈ 6) with conc. HCL and loaded onto a DOWEX 1w50 strong cation exchange resin. The column was eluted with water → 10% NH₄OH and the fractions were analysed by ESMS⁺. The fractions were combined and lyophilised to yiled the *title compound* as a dark orange oil (26 mg, 33 µmol, 55%), which has been used in a complexation reaction immediately, *m*/*z* (ESMS⁻) 779 (M - 1)

### (v) Preparation of Eu-Complex

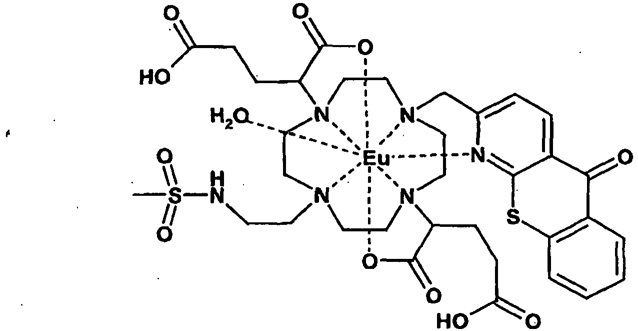

4-[(1-Azathioxanthone)-2-methyl]-10-[methylsulfonylamino)ethyl]-1,7bis(α-glutarate)-1,4,7,10-tetraazacyclododecane (25 mg, 32 µmol) was added to Eu(CH₃CO₂)₃ (1.1 eq., 15 mg) and the solids dissolved in a H₂O (2 mL). The pH was carefully adjusted to 5 by addition of acetic acid and the reaction left to stir at 70 ° C for 72 h. After the reaction was cooled to room temperature, the solvents were removed under reduced pressure, the remaining residue was dissolved in 5 mL H₂O. The pH was then adjusted carefully to 10 by addition of conc. NaOH solution (in order to get rid of the excess europium as Eu(OH)₃) . This resulted in a white precipitate that was removed via a fine syringe filter. The pH was adjusted back to neutral and lyophilised to give a bright yellow solid. *m*/*z* (HRMS⁺) 927.1564 (C₂₃₄H₄₂O₁₁N₆S₂Eu requires 927.1565)

## Claims

1. A luminescent lanthanide complex comprising a lanthanide (III) ion with a xanthone or thiaxanthone sensitising moiety, coordinated to the lanthanide ion by the nitrogen atom of an integral pyridyl group or a related group able to bind the lanthanide ion, the complex having any one of the following structures 4 and 5: 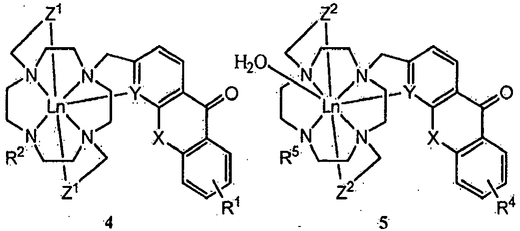 (wherein:
R¹ is H, alkyl, aralkyl, CO₂R³, CONHR³;
R² is H, alkyl or aralkyl;
R³ is alkyl, aralkyl or aryl, optionally substituted;
R⁴ is H, alkyl, aralkyl, CO₂R⁶;
R⁵ is CH₂CH₂NHSO₂R⁶;
R⁶ is alkyl, *p*-R⁴-Ph, *p*-OMe-Ph, *p*-CF₃-Ph;
X is O or S;
Y is N N-oxide or C-OH
Z¹ is CO₂, PR¹O₂, pO₃²⁻, CONHR³;
Z² is CO₂, PR⁴O₂, PO₃²⁻, CONHR⁴ and
Ln is a lanthanide (III) ion).

2. The complex as claimed in claim 1 wherein Y is nitrogen.

3. The complex as claimed in claim 1 or claim 2 wherein X is sulfur.

4. The complex as claimed in claim 1 which comprises 1-(2'-[1-azathioxanthone]methyl)-4,10-*bis*[(S,S)-ethyl-2"-carbamoylmethyl-3-phenylpropanoate]-1,4,7,10-tetra-azacyclododecane.

5. The complex as claimed in claim 1 which comprises 4-[(1-azathioxanthone)-2-methyl]-1;7-bis(carboxylmethyl)-1,4,7,10-tetra-azacyclododecane, 4-[(1-azathioxanthone)-2-methyl]-10-[methylsulfonylamino)ethyl]-1,7-bis (carboxylmethyl)-1,4,7,10-tetraazacyclododecane or 4-[(1-azathioxanthone)-2-methyl]-10-[methylsulfonylamino)ethyl]-1,7-bis(α-glutarate)-1,4,7,10-tetraazacyclododecane.

6. The complex as claimed in any one preceding claim where the lanthanide ion is europium or terbium.

7. The complex as claimed in claim 6 wherein the lanthanide ion is europium.

8. A method of modulating a complex as defined in any one of claims 1 to 7 comprising reacting the complex with a ligand.

9. The method of claim 8 wherein said ligand is an anion.

10. The method of claim 9 wherein the anion is a phosphonoanion, citrate, lactate or hydrogencarbonate:

11. The method of claim 10 wherein the anion is citrate.

12. The method of claim 11 wherein the method is used to signal variation in the concentration of the citrate anion.

13. A method of modulating a complex as defined in any one of claims 1 to 7 comprising subjecting the solution to complex to a pH change.

14. The method of claim 13 wherein the pH is varied over the range 3 to 9.

15. The method of claim 13 wherein the pH is varied over the range 4.5 to 8.

16. The method of any one of claims 13 to 15 wherein the method is used to monitor local pH changes using monitoring local pH changes in real time in a ratiometric analytical method.

17. The method of claim 16 wherein the analytical method is conducted as part of an *in vitro, in cellulo* or *in vivo* assay or application.

18. The method of claim 17 wherein said assay or application the analytical method is conducted as part of an *in vitro* assay or application.

19. The method of claim 17 or claim 18 wherein said assay or application involves time-resolved luminescence using microscopy or spectroscopy.

20. A compound having any one of the following formulae 1 and 2: 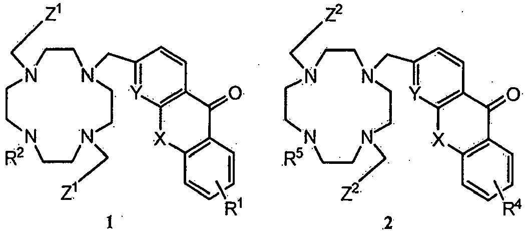 (wherein R¹, R², X, Y, Z¹ and, Z² are as defined in claim 1).

21. The compound of claim 20, wherein Y is nitrogen and/or X is sulfur.

22. The compound of claim 20 which is 1-(2'-[1-azathioxanthone]methyl)-4,10-*bis*[(S,S)-ethyl-2"-carbamoylmethyl-3-phenylpropanoate]-1,4,7,10-tetra-azacyclododecane.

23. The compound of claim 20 which is 4-[(1-azathioxanthone)-2-methyl]-1,7-bis(carboxylmethyl)-1,4,7,10-tetra-azacyclododecane, 4-[(1-azathioxanthone)-2-methyl]-10-[methylsulfonylamino)ethyl]-1,7-bis (carboxylmethyl)-1,4,7,10-tetraazacyclododecane or 4-[(1-azathioxanthone)-2-methyl]-10-[methylsulfonylamino)ethyl]-1,7-bis(a-glutarate)-1,4,7,10-tetraazacyclododecane.

## Patentansprüche

1. Lumineszenter Lanthanoidkomplex, umfassend ein Lanthanoid(III)ion mit einem sensibilisierenden Xanthon- oder Thiaxanthonteil, koordiniert an das Lanthanoidion durch das Stickstoffatom einer integralen Pyridylgruppe oder einer verwandten Gruppe mit der Fähigkeit, das Lanthanoidion zu binden, wobei der Komplex eine der folgenden Strukturen 4 und 5 aufweist: 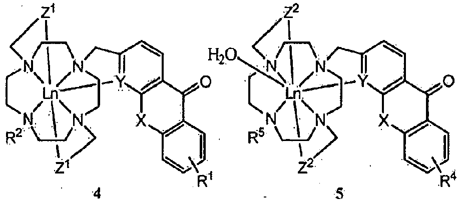 (wobei:
R¹ H, Alkyl, Aralkyl, CO₂R³, CONHR³ ist;
R² H, Alkyl oder Aralkyl ist;
R³ Alkyl, Aralkyl oder Aryl, wahlweise substituiert, ist;
R⁴ H, Alkyl, Aralkyl, CO₂R⁶ ist;
R⁵ CH₂CH₂NHSO₂R⁶ ist;
R⁶ Alkyl, *p*-R⁴-Ph, *p*-OMe-Ph, *p*-CF₃-Ph ist;
X O oder S ist;
Y N, N-Oxid oder C-OH ist
Z¹ CO₂⁻, PR¹O₂⁻, PO₃²⁻, CONHR³ ist;
Z² CO₂ -, PR⁴O₂⁻, PO₃²⁻, CONHR⁴ ist und
Ln ein Lanthanoid(III)ion ist).

2. Komplex nach Anspruch 1, wobei Y Stickstoff ist.

3. Komplex nach Anspruch 1 oder Anspruch 2, wobei X Schwefel ist.

4. Komplex nach Anspruch 1, der 1-(2'-[1-Azathioxanthon]methyl)-4,10-*bis*[(S,S)-ethyl-2"-carbamoylmethyl-3-phenylpropanoat]-1,4,7,10-tetra-azacyclododecan umfasst.

5. Komplex nach Anspruch 1, der 4-[(1-Azathioxanthon)-2-methyl]-1,7-bis(carboxylmethyl)-1,4,7,10-tetra-azacyclododecan, 4-[(1-Azathioxanthon)-2-methyl]-10-[methylsulfonylamino)ethyl]-1,7-bis(carboxylmethyl)-1,4,7,10-tetraazacyclododecan oder 4-[(1-Azathioxanthon)-2-methyl]-10-[methylsulfonylamino)ethyl]-1,7-bis(α-glutarat)-1,4,7,10-tetraazacyclododecan umfasst.

6. Komplex nach einem der vorstehenden Ansprüche, wobei das Lanthanoidion Europium oder Terbium ist.

7. Komplex nach Anspruch 6, wobei das Lanthanoidion Europium ist.

8. Verfahren zum Modulieren eines Komplexes gemäß Definition in einem der Ansprüche 1 bis 7, umfassend Umsetzen des Komplexes mit einem Liganden.

9. Verfahren nach Anspruch 8, wobei der Ligand ein Anion ist.

10. Verfahren nach Anspruch 9, wobei das Anion ein Phosphonoanion, Citrat, Lactat oder Hydrogencarbonat ist.

11. Verfahren nach Anspruch 10, wobei das Anion Citrat ist.

12. Verfahren nach Anspruch 11, wobei das Verfahren verwendet wird, um eine Veränderung der Konzentration des Citratanions zu signalisieren.

13. Verfahren zum Modulieren eines Komplexes gemäß Definition in einem der Ansprüche 1 bis 7, das umfasst, dass die zu komplexierende Lösung einer pH-Wert-Änderung ausgesetzt wird.

14. Verfahren nach Anspruch 13, wobei der pH-Wert über den Bereich 3 bis 9 variiert wird.

15. Verfahren nach Anspruch 13, wobei der pH-Wert über den Bereich 4,5 bis 8 variiert wird.

16. Verfahren nach einem der Ansprüche 13 bis 15, wobei das Verfahren verwendet wird, um lokale pH-Wert-Änderungen mittels Überwachung lokaler pH-Wert-Änderungen in Echtzeit in einem ratiometrischen analytischen Verfahren zu überwachen.

17. Verfahren nach Anspruch 16, wobei das analytische Verfahren als Teil eines/-r *In-vitro-, In-cellulo-* oder *In*-*vivo*-Assays oder -Anwendung ausgeführt wird.

18. Verfahren nach Anspruch 17, wobei der Assay oder die Anwendung des analytischen Verfahrens als Teil eines/-r *In-vitro-*Assays oder -Anwendung ausgeführt wird.

19. Verfahren nach Anspruch 17 oder Anspruch 18, wobei der Assay oder die Anwendung zeitaufgelöste Lumineszenz unter Verwendung von Mikroskopie oder Spektroskopie involviert.

20. Verbindung mit einer der folgenden Formeln 1 und 2: 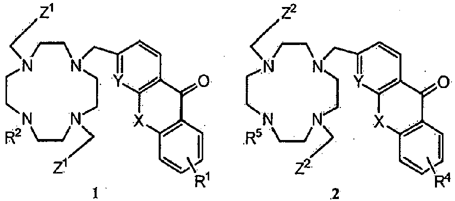 (wobei R¹, R², X, Y, Z¹ und Z² wie in Anspruch 1 definiert sind).

21. Verbindung nach Anspruch 20, wobei Y Stickstoff ist und/oder X Schwefel ist.

22. Verbindung nach Anspruch 20, die 1-(2'-[1-Azathioxanthon]methyl)-4,10-*bis*[(S,S)-ethyl-2"-carbamoylmethyl-3-phenylpropanoat]-1,4,7,10-tetra-azacyclododecan ist.

23. Verbindung nach Anspruch 20, die 4-[(1-Azathioxanthon)-2-methyl]-1,7-bis(carboxylmethyl)-1,4,7,10-tetra-azacyclododecan, 4-[(1-Azathioxanthon)-2-methyl]-10-[methylsulfonylamino)ethyl]-1,7-bis(carboxylmethyl)-1,4,7,10-tetraazacyclododecan oder 4-[(1-Azathioxanthon)-2-methyl]-10-[methylsulfonylamino)ethyl]-1,7-bis(α-glutarat)-1,4,7,10-tetraazacyclododecan ist.

## Revendications

1. Complexe de lanthanide luminescent comprenant un ion lanthanide (III) avec un fragment sensibilisant xanthone ou thiaxanthone, coordonné à l'ion lanthanide par l'atome d'azote d'un groupe pyridyle formant un tout unitaire ou un groupe apparenté apte à se lier à l'ion lanthanide, le complexe ayant l'une quelconque des structures 4 et 5 suivantes: 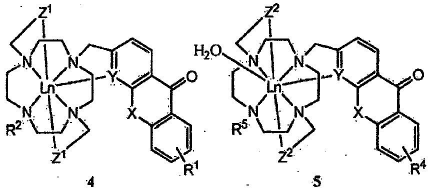 (dans lesquelles:
R¹ est H, alkyle, aralkyle, CO₂R³, CONHR³;
R² est H, alkyle ou aralkyle;
R³ est alkyle, aralkyle ou aryle, facultativement substitué;
R⁴ est H, alkyle, aralkyle, CO₂R⁶;
R⁵ est CH₂CH₂NHSO₂R⁶;
R⁶ est alkyle, *p*-R⁴-Ph, *p*-OMe-Ph, *p*-CF₃-Ph;
X est O ou S;
Y est N, N-oxyde ou C-OH
Z¹ est CO₂⁻, PR¹O₂⁻, PO₃²⁻, CONHR³;
Z² est CO₂⁻, PR⁴O₂⁻, PO₃²⁻, CONHR⁴ et
Ln est un ion lanthanide (III)).

2. Complexe selon la revendication 1, dans lequel Y est de l'azote.

3. Complexe selon la revendication 1 ou la revendication 2, dans lequel X est du soufre.

4. Complexe selon la revendication 1, qui comprend du 1-(2'-[1-azathioxanthone]méthyl)-4,10-*bis*[(S,S)-éthyl-2"-carbamoylméthyl-3-phénylpropanoate]-1,4,7,10-tétra-azacyclododécane.

5. Complexe selon la revendication 1, qui comprend du 4-[(1-azathioxanthone)-2-méthyl]-1,7-bis(carboxylméthyl)-1,4,7,10-tétra-azacyclododécane, du 4-[(1-azathioxanthone)-2-méthyl]-10-[méthylsulfonylamino)éthyl]-1,7-bis(carboxylméthyl)-1,4,7,10-tétraazacyclododécane ou du 4-[(1-azathioxanthone)-2-méthyl]-10-[méthylsulfonylamino)éthyl]-1,7-bis(a-glutarate)-1,4,7,10-tétraazacyclododécane.

6. Complexe selon l'une quelconque des revendications précédentes, dans lequel l'ion lanthanide est l'europium ou le terbium.

7. Complexe selon la revendication 6, dans lequel l'ion lanthanide est l'europium.

8. Procédé de modulation d'un complexe tel que défini dans l'une quelconque des revendications 1 à 7, comprenant la réaction du complexe avec un ligand.

9. Procédé selon la revendication 8, dans lequel ledit ligand est un anion.

10. Procédé selon la revendication 9, dans lequel l'anion est un phosphonoanion, un citrate, un lactate ou un hydrogénocarbonate.

11. Procédé selon la revendication 10, dans lequel l'anion est un citrate.

12. Procédé selon la revendication 11, lequel procédé est utilisé pour signaler une variation de la concentration de l'anion citrate.

13. Procédé de modulation d'un complexe tel que défini dans l'une quelconque des revendications 1 à 7, comprenant la soumission de la solution qui doit être complexée à un changement de pH.

14. Procédé selon la revendication 13, dans lequel on fait varier le pH dans la gamme de 3 à 9.

15. Procédé selon la revendication 13, dans lequel on fait varier le pH dans la gamme de 4,5 à 8.

16. Procédé selon l'une quelconque des revendications 13 à 15, lequel procédé est utilisé pour suivre des changements de pH locaux en utilisant un suivi des changements de pH locaux en temps réel dans un procédé analytique ratiométrique.

17. Procédé selon la revendication 16, dans lequel le procédé analytique est mis en oeuvre en tant que partie d'un essai ou d'une application *in vitro, in cellulo* ou *in vivo.*

18. Procédé selon la revendication 17, dans lequel ledit essai ou ladite application du procédé analytique est mis(e) en oeuvre en tant que partie d'un essai ou d'une application *in vitro.*

19. Procédé selon la revendication 17 ou la revendication 18, dans lequel ledit essai ou ladite application fait intervenir une luminescence à résolution temporelle en utilisant une microscopie ou une spectroscopie.

20. Composé ayant l'une quelconque des formules 1 et 2 suivantes: 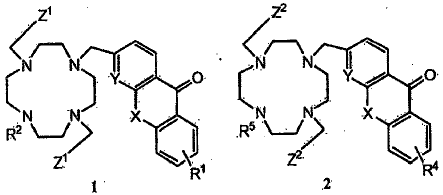 (dans lesquelles R¹, R², X, Y, Z¹ et Z² sont tels que définis dans la revendication 1).

21. Composé selon la revendication 20, dans lequel Y est de l'azote et/ou X est du soufre.

22. Composé selon la revendication 20, qui est le 1-(2'-[1-azathioxanthone]méthyl)-4,10-*bis*[(S,S)-éthyl-2"-carbamoylméthyl-3-phénylpropanoate]-1,4,7,10-tétra-azacyclododécane.

23. Composé selon la revendication 20, qui est le 4-[(1-azathioxanthone)-2-méthyl]-1,7-bis(carboxylméthyl)-1,4,7,10-tétra-azacyclododécane, le 4-[(1-azathioxanthone)-2-méthyl]-10-[méthylsulfonylamino)éthyl]-1,7-bis(carboxylméthyl)-1,4,7,10-tétraazacyclododécane ou le 4-[(1-azathioxanthone)-2-méthyl]-10-[méthylsulfonylamino)éthyl]-1,7-bis(α-glutarate)-1,4,7,10-tétraazacyclododécane.
